# NEW EUROPEAN PATENT SPECIFICATION

(11) **EP 2 442 799 B2**
(45) Date of publication and mention of the opposition decision: **22.05.2019**
(45) Mention of the grant of the patent: 07.09.2016
(21) Application number: 10725746.1
(22) Date of filing: 18.06.2010
(51) Int. Cl.: A61K 9/20, A61K 31/5377

(54) **Solid pharmaceutical composition comprising rivaroxaban**
Feste pharmazeutische Zusammensetzung mit Rivaroxaban
Composition pharmaceutique solide comprenant du rivaroxaban

(30) Priority: 18.06.2009 EP 09163177; 12.03.2010 EP 10156445
(43) Date of publication of application: 25.04.2012
(73) Proprietor: KRKA, tovarna zdravil, d.d., Novo mesto, 8501 Novo mesto (SI)
(72) Inventor: SEDMAK, Gregor, 1000 Ljubljana (SI); VRECER, Franc, 8351 Straza pri Novem mestu (SI); MEZNAR, Klavdija, 8000 Novo mesto (SI); TROST, Sabina, 1330 Kocevje (SI); BUKOVEC, Polona, 8000 Novo mesto (SI); HVALEC, Miran, 8000 Novo mesto (SI)
(74) Representative: Uexküll & Stolberg
(86) International application number: PCT/EP2010/058680
(87) International publication number: WO 2010/146179

(56) References cited:
- WO-A-2005/060940
- WO-A-2007/039122
- WO-A1-2010/003641
- WO-A2-2010/017948
- US-A1- 2007 026 065

## Description

The disclosure relates to a solid pharmaceutical composition comprising rivaroxaban. It further relates to a process for the preparation of a pharmaceutical composition comprising rivaroxaban.

### Background of the invention

Rivaroxaban is the INN of the anticoagulant compound 5-chloro-N-{[(5S)-2-oxo-3-[4-(3-oxomorpholin-4-yl)phenyl]oxazolidin-5-yl]methyl}thiophene-2-carboxamide, which was originally disclosed in WO 01/47919 A1. Rivaroxaban is a small molecule inhibitor of blood coagulation factor Xa and is used in the prophylaxis and treatment of thromboembolic diseases such as heart attack, angina pectoris, reocclusion and restenosis following angioplasty or bypass, cerebral apoplexy, transient ischemic attack, peripheral arterial obstructive diseases, pulmonary embolism and venous thrombosis.

Rivaroxaban is characterized by a very low solubility in water (about 7 mg/L) and many organic solvents as well as a high melting point (about 230 °C). The low solubility is particularly problematic with respect to an oral application, which results in a poor bioavailability.

A number of approaches have been pursued in order to increase the oral bioavailability of rivaroxaban.

WO 2005/060940 A2 describes a process for the preparation of a solid pharmaceutical composition comprising rivaroxaban in hydrophilized form, wherein a granulate prepared by moist granulation of rivaroxaban with a hydrophilic binder is converted to the pharmaceutical composition. Tablets comprising hydrophilized active agent are shown to exhibit increased bioavailability as compared with tablets comprising non-hydrophilized active agent. However, the moist granulation processes used for hydrophilization of the active agent are complex, require special equipment and are difficult to use on an industrial scale. Moreover, they require great amounts of energy for the evaporation of granulation liquid.

WO 2007/039122 A2 discloses solid pharmaceutical dosage forms comprising rivaroxaban in amorphous form and/or in the form of thermodynamically metastable crystal modifications. These dosage forms are described as showing an increased rate of dissolution as well as increased solubility. However, obtaining rivaroxaban in amorphous form or in the form of thermodynamically metastable crystal modifications is problematic because it involves dissolving and/or melting the active agent. In particular, dissolution processes require excessively large amounts of solvent because of the poor solubility of rivaroxaban in water and pharmaceutically acceptable organic solvents like ethanol or acetone. Due to the high melting point of rivaroxaban, processes involving melting the active agent result in significant decomposition thereof.

WO 2007/039132 A1 discloses certain polymorphous forms as well as the amorphous form of rivaroxaban.

US 2007/026065 A1 discloses a process comprising hot melt extrusion of rivaroxaban and an excipient, performed at a temperature exceeding 150°C.

It is an object of the present invention to provide a pharmaceutical composition comprising rivaroxaban avoiding these drawbacks and which is not only bioequivalent with the known formulations of rivaroxaban, but which can also be prepared from inexpensive excipients and can be prepared using a simple and economical process that is better suited for industrial application.

### Description of the invention

The invention relates to a process for the preparation of a solid pharmaceutical composition comprising rivaroxaban, or a pharmaceutically acceptable salt or solvate thereof, the process comprising
(a) providing a mixture comprising rivaroxaban, or a pharmaceutically acceptable salt or solvate thereof, and at least one pharmaceutically acceptable excipient comprising (i) hot melt granulation of rivaroxaban with at least one excipient and optionally mixing the obtained granulate with additional excipients or (ii) hot melt extrusion of rivaroxaban with at least one excipient, milling the extrudate and optionally mixing the milled extrudate with additional excipients; and
(b) converting said mixture to a solid pharmaceutical composition, preferably by compressing the mixture to give a tablet;
wherein said process is performed at a temperature of not more than 150 °C and wherein the at least one excipient used in step (a) comprises at least one low-melting binder having a melting point below 120 °C which is selected from poloxamers, polyethylene glycols having an average molecular weight of below 15,000, macrogol glycerides and esters of glycerol with fatty acids having 10 to 24 carbon atoms.

In one aspect a solid pharmaceutical composition is disclosed, comprising rivaroxaban, or a pharmaceutically acceptable salt or solvate thereof, and at least one pharmaceutically acceptable excipient. It is preferred that the solid pharmaceutical composition is in the form of a tablet or capsule or in the form of pellets or granules.

The composition comprises rivaroxaban, or a pharmaceutically acceptable salt or solvate thereof, as active ingredient. Generally, rivaroxaban can be used in any crystalline, partly crystalline or amorphous form or modification. Preferably, rivaroxaban is used in the crystalline modification in which it is obtained according to the processes disclosed in WO 01/47919 A1, J. Med. Chem 48, 5900-5908 (2005) or WO 2007/039132. It is further preferred that the rivaroxaban is in non-hydrophilized form.

Preferably the rivaroxaban is in micronized form with d₉₀ < 60 µm, more preferably d₉₀ < 40 µm, and most preferably d₉₀ < 30 µm. As used herein d₉₀ < x means that at least 90 % by volume of the particles have a particle size below x. The particle size can be determined by laser light scattering for instance using a Malvern Mastersizer Apparatus MS 2000 equipped with a Hydro S dispersion unit using purified water as the dilution medium. Micronized rivaroxaban can be obtained for instance by single or multi-stage micronization in the dry or wet state. Dry state milling can be achieved by using dry mills, such as cutting mills, pin/cage mills, hammer mills, jet mills, fluidized bed jet mills, ball mills and roller mills. Wet state milling can be achieved by using wet mills, such as colloid mills, ultra sound processors, high pressure homogenizers, wet pearl mills and agitated ball mills.

The composition preferably comprises 0.1 to 60 wt.-%, particularly 0.5 to 40 wt.-%, more preferably 1 to 20 wt.-%, most preferably 5 to 15 wt.-% of rivaroxaban or a pharmaceutically acceptable salt or solvate thereof. All percentages given herein are by weight of the total composition unless specifically stated otherwise.

The term "pharmaceutical acceptable excipient" as used herein refers to additives useful for converting pharmacologically active compounds into pharmaceutical dosage forms which are suitable for administration to patients. Suitable excipients include fillers, binders, disintegrants, surfactants, lubricants, glidants and coloring agents. Other pharmaceutically acceptable excipients can also be included.

The composition typically comprises at least one filler. Suitable fillers include monosaccharides and oligosaccharides such as glucose, fructose, saccharose, lactose (anhydrous and monohydrate), raffinose, trehalose and dextrates, sugar alcohols such as mannitol, sorbitol, maltitol, xylitol and lactitol, compressible sugar, microcrystalline cellulose, powdered cellulose, siliconized microcrystalline cellulose, silicon dioxide, crospovidone, croscarmellose sodium, calcium hydrogen phosphate, calcium carbonate, calcium lactate and mixtures thereof. Microcrystalline cellulose and water-soluble fillers are preferred. Water-soluble fillers are particularly preferred.

The composition preferably comprises 20 to 99 wt.-%, particularly 50 to 95 wt.-%, more preferably 70 to 90 wt.-%, most preferably 80 to 90 wt.-% of filler. According to another embodiment, the composition comprises 20 to 99 wt.-%, particularly 30 to 90 wt.-%, more preferably 40 to 70 wt.-% and most preferably 50 to 60 wt.-% of filler.

The term "water-soluble filler" as used herein refers to a filler having a solubility in water at 25 °C of at least 0.03 g/L. Examples of water-soluble fillers include monosaccharides and oligosaccharides such as glucose, fructose, saccharose, lactose (anhydrous and monohydrate), raffinose, trehalose and dextrates, sugar alcohols such as mannitol, sorbitol, maltitol, xylitol and lactitol. Lactose, mannitol, sorbitol or xylitol are particularly preferred. It is particularly preferred that the composition comprises at least 35 wt.-%, particularly 35 to 90 wt.-%, more particularly 35 to 80 wt.-%, preferably 40 to 70 wt.-%, more preferably 40 to 60 wt.-% and most preferably 45 to 55 wt.-% of at least one water-soluble filler.

The composition can also include at least one water-insoluble filler. The term "water-insoluble filler" as used herein refers to a filler having a solubility in water at 25 °C of less than 0.03 g/L. Examples of water-insoluble fillers include microcrystalline cellulose, powdered cellulose, siliconized microcrystalline cellulose, silicon dioxide, crospovidone, croscarmellose sodium, calcium hydrogen phosphate, calcium carbonate, calcium lactate and mixtures thereof. Microcrystalline cellulose and calcium hydrogen phosphate are particularly preferred. It is preferred that the composition comprises 5 to 50 wt.-%, more preferably 10 to 40 wt.-% and most preferably 30 to 40 wt.-% of at least one water-insoluble filler. According to another embodiment, the composition comprises 1 to 50 wt.-%, preferably 5 to 50 wt.-%, more preferably 8 to 30 wt.-% and most preferably 10 to 20 wt.-% of at least one water-insoluble filler.

It is further preferred that the composition comprises water-soluble filler and water-insoluble filler in a weight ratio of 1:1 to 10:1, particularly 1:1 to 5:1, and most preferably 1.1:1 to 1.5:1. According to another embodiment, the filler comprises water-soluble filler and water-insoluble filler in a weight ratio of 1:10 to 30:1, particularly 1:1 to 20:1, preferably 1:1 to 10:1, more preferably 2:1 to 8:1, and most preferably 3:1 to 5:1.

Suitable binders include povidone (polyvinylpyrrolidone), copovidone (vinylpyrrolidone-vinylacetate copolymer), microcrystalline cellulose, powdered cellulose, crystalline cellulose, siliconized microcrystalline cellulose, cellulose derivatives such as hydroxymethylcellulose, hydroxyethylcellulose, hydroxypropylcellulose and hydroxypropylmethylcellulose, low-substituted hydroxypropylcellulose, starch, pregelatinized starch, polymethacrylates, compressible sugars, sucrose and sugar alcohols such as mannitol, sorbitol, maltitol and xylitol and mixtures thereof. Preferred are povidone, copovidone, mannitol and sorbitol.

The binder comprises at least one low-melting binder having a melting point below 120 °C, particularly below 100 °C and most preferably below 80 °C which is selected from poloxamers, polyethyleneglycols having an average molecular weight of below 15,000 and particularly in the range of 1,500 to 10,000, macrogol glycerides such as Gelucire® and esters of glycerol with fatty acids having 10 to 24 carbon atoms such as glycerol monostearate and glycerol behenate. Binders having a melting point below 80°C and/or having surfactant properties are particularly preferred. As used herein, the term "having surfactant properties" refers to a substance decreasing the surface tension of water by at least 20 % in comparison to pure water at 25 °C when used at a concentration of 0.1 g/L or less. Low temperature-melting binders and binders having surfactant properties are particularly suitable for use in hot melt processes.

The composition preferably comprises 0.5 to 40 wt.-%, particularly 1 to 20 wt.-%, more preferably 2 to 10 wt.-%, most preferably 3 to 7 wt.-% of binder. According to another embodiment, the composition comprises 0 to 40 wt.-%, particularly 1 to 30 wt.-%, more preferably 5 to 25 wt.-%, and most preferably 10 to 20 wt.-% of low temperature-melting binder.

The term "disintegrant" as used herein refers to any material that has wicking and/or swelling properties when it comes in contact with water. Suitable disintegrants include povidone, crospovidone, starch, pregelatinized starch, sodium starch glycolate, hydroxypropyl starch, microcrystalline cellulose, carboxymethylcellulose sodium or calcium, croscarmellose, polacrilin potassium, low-substituted hydroxypropylcellulose, sodium or calcium alginate, docusate sodium, methylcellulose, agar, guar gum, chitosan, alginic acid and mixtures thereof. Preferred are crospovidone and croscarmellose. The composition preferably comprises 1 to 20 wt.-%, particularly 2 to 10 wt.-%, more preferably 3 to 8 wt.-%, and most preferably 4 to 7 wt.-% of disintegrant.

Suitable surfactants include anionic surfactants such as sodium lauryl sulfate and docusate sodium, cationic surfactants such as cetrimide, ampholytic surfactants such as N-dodecyl-N,N-dimethylbetaine, non-ionic surfactants such as sorbitan fatty acid esters (e.g. Spans®), polysorbates (e.g. Tweens®), polyoxyethylene alkyl ethers (e.g. Brij®, Volpo®), poloxamers (e.g. Lutrol®), medium-chain triglycerides (e.g. Miglyol®), polyoxylglycerides (e.g. Gelucire®), polyoxyethylene castor oil derivatives (e.g. Cremophor®) and mixtures thereof.

In particular, the surfactant may be a liquid or semi-solid waxy surfactant. As used herein, the term liquid or semi-solid waxy surfactant refers to a surfactant having a melting point of less then 160 °C, preferably less then 120 °C, more preferably less then 80 °C and most preferably less then 65 °C. Examples of liquid or semi-solid waxy surfactants include docusate sodium and non-ionic surfactants such as sorbitan fatty acid esters (e.g. Spans®), polysorbates (e.g. Tweens®), polyoxyethylene alkyl ethers (e.g. Brij®, Volpo®), poloxamers (e.g. Lutrol®), medium-chain triglycerides (e.g. Miglyol®), polyoxylglycerides (e.g. Gelucire®), polyoxyethylene castor oil derivatives (e.g. Cremophor®) and mixtures thereof.

Preferably, a liquid or semi-solid waxy surfactant is used in the form of a surfactant/filler dispersion. The surfactant/filler dispersion preferably comprises at least one surfactant and at least one filler in a ratio of from 10:1-1:100, particularly 5:1 - 1:50, preferably 2:1 - 1:10, more preferably 1:1 - 1:5, most preferably 1:2 - 1:10.

It has been found that using a liquid or semi-solid waxy surfactant in the form of a surfactant/filler dispersion is suitable to overcome the problems typically associated with the use of such surfactants in the preparation of e.g. solid tablets formulations.

Preferred surfactants include sodium lauryl sulfate, docusate sodium and liquid or semi-solid waxy surfactants such as polysorbates and poloxamers. Sodium lauryl sulfate is particularly preferred.

The composition preferably comprises 0 to 50 wt.-%, particularly 0.5 to 30 wt.-%, more preferably 0.5 to 20 wt.-%, particularly 1 to 10 wt.-%, more preferably 1.5 to 5 wt.-%, and most preferably 2 to 3 wt.-% of surfactant. According to another embodiment, the composition comprises 0 to 30 wt.-%, particularly 0.2 to 5 wt.-%, more preferably 0.5 to 3 wt.-%, and most preferably 1 to 2 wt.-% of surfactant. According to another embodiment, the composition preferably comprises 0 to 50 wt.-%, particularly 0.5 to 30 wt.-%, more preferably 1 to 20 wt.-%, particularly 2 to 15 wt.-%, more preferably 3 to 10 wt.-%, and most preferably 4 to 8 wt.-% of surfactant, particularly liquid or semi-solid waxy surfactant. According to another embodiment, the composition preferably comprises 0 to 50 wt.-%, particularly 0.5 to 30 wt.-%, more preferably 1 to 20 wt.-%, particularly 2 to 15 wt.-%, more preferably 5 to 12 wt.-%, and most preferably 8 to 10 wt.-% of surfactant, particularly liquid or semi-solid waxy surfactant.

Suitable lubricants include fatty acids such as stearic acid, palmitic acid and oleic acid, fatty acid salts such as magnesium stearate, magnesium palmitate and magnesium oleate, hydrogenated vegetable oil, hydrogenated castor oil, talc, sodium stearyl fumarate, macrogols and mixtures thereof. Stearic acid, magnesium stearate and hydrogenated vegetable oil are particularly preferred. The composition preferably comprises 0.1 to 10 wt.-%, particularly 0.25 to 5 wt.-%, and more preferably 0.5 to 2 wt.-% of lubricant.

Suitable glidants include colloidal silicon dioxide and magnesium trisilicate. The composition preferably comprises 0.1 to 10 wt.-%, particularly 0.25 to 5 wt.-%, and more preferably 0.5 to 2 wt.-% of glidant.

Suitable coloring agents include dyes and pigments such as iron oxide and titanium oxide. The composition preferably comprises 0.001 to 1 wt.-%, particularly 0.01 to 0.5 wt.-%, and more preferably 0.05 to 0.2 wt.-% of coloring agent.

The composition can also include an acid which may serve to improve the solubility of the active ingredient. Preferred acids are organic acids, in particular citric acid. The acid is preferably present in an amount of 0 to 15 wt.-%, in particular 1 to 12 wt.-%.

According to one aspect, the composition comprises
(a) 0.1 to 60 wt.-%, particularly 0.5 to 40 wt.-%, more preferably 1 to 20 wt.-%, most preferably 5 to 15 wt.-% of rivaroxaban or a pharmaceutically acceptable salt or solvate thereof;
(b) 30 to 99 wt.-%, particularly 50 to 95 wt.-%, more preferably 70 to 90 wt.-%, more preferably 80 to 90 wt.-% of filler, most preferably 80 to 87 wt.-% of filler;
(c) 0 to 40 wt.-%, particularly 1 to 20 wt.-%, more preferably 2 to 10 wt.-%, most preferably 3 to 7 wt.-% of binder;
(d) 0 to 20 wt.-%, particularly 2 to 10 wt.-%, preferably 3 to 8 wt.-%, more preferably 4 to 7 wt.-%, more preferably 2 to 5 wt.-%, most preferably 2 to 4 wt.-% of disintegrant;
(e) 0 to 50 wt.-%, particularly 0.5 to 30 wt.-%, more preferably 0.5 to 20 wt.-%, particularly 1 to 10 wt.-%, more preferably 1.5 to 5 wt.-%, most preferably 2 to 3 wt.-% of surfactant;
(f) 0 to 10 wt.-%, particularly 0.25 to 5 wt.-%, more preferably 0.5 to 2 wt.-% of lubricant;
(g) 0 to 10 wt.-%, particularly 0.25 to 5 wt.-%, more preferably 0.5 to 2 wt.-% of glidant;
(h) 0 to 1 wt.-%, particularly 0.01 to 0.5 wt.-%, more preferably 0.05 to 0.2 wt.-% of coloring agent; and
(i) 0 to 15 wt.-%, and particularly 1 to 12 wt.-% of organic acid.

Compositions according to this embodiment are particularly suitable to be prepared by using dry mixing, compression into tablets using liquid or semi-solid waxy surfactants, dry granulation, co-milling or a combination thereof.

According to an embodiment, the composition comprises
(a) 0.1 to 60 wt.-%, particularly 0.5 to 40 wt.-%, more preferably 1 to 20 wt.-%, most preferably 5 to 15 wt.-% of rivaroxaban or a pharmaceutically acceptable salt or solvate thereof;
(b) 20 to 99 wt.-%, particularly 30 to 90 wt.-%, more preferably 40 to 70 wt.-%, most preferably 50 to 60 wt.-% of filler;
(c) 0 to 40 wt.-%, particularly 1 to 30 wt.-%, more preferably 5 to 25 wt.-%, most preferably 10 to 20 wt.-% of binder;
(d) 0 to 20 wt.-%, particularly 2 to 10 wt.-%, more preferably 3 to 8 wt.-%, most preferably 4 to 7 wt.-% of disintegrant;
(e) 0 to 30 wt.-%, particularly 0.2 to 5 wt.-%, more preferably 0.5 to 3 wt.-%, most preferably 1 to 2 wt.-% of surfactant;
(f) 0 to 10 wt.-%, particularly 0.25 to 5 wt.-%, more preferably 0.5 to 2 wt.-% of lubricant;
(g) 0 to 10 wt.-%, particularly 0.25 to 5 wt.-%, more preferably 0.5 to 2 wt.-% of glidant;
(h) 0 to 1 wt.-%, particularly 0.01 to 0.5 wt.-%, more preferably 0.05 to 0.2 wt.-% of coloring agent;
(i) 0 to 15 wt.-%, and particularly 1 to 12 wt.-% of organic acid.

Compositions according to this embodiment are particularly suitable to be prepared by using hot melt processes such as hot melt granulation or hot melt extrusion.

The composition is optionally film coated. Film coating can be used to provide for improved surface smoothness and color, increased chemical and physical stability of the active agent due to reduced permeability for gases such as oxygen and/or water vapor, less disintegration of the solid composition in acidic medium resulting in decreased gastrointestinal side effects, and easier swallowing of tablet. The film coating comprises coating materials generally known in the art. Suitable coating materials include polymers such as hydroxypropylcellulose, hydroxypropylmethylcellulose, ethylcellulose, povidone and copovidone, graft copolymers of polyethyleneglycol and polyvinyl alcohol (Kollicoat IR), shellac, polymers of methacrylic acid or methacrylic acid esters, methacrylic acid-methyl acrylate copolymers, polyvinyl alcohol, plasticizers such as propylene glycol, polyethylene glycol, glycerol triacetate, triethyl citrate, antitacking agents such as talc, glycerol monostearate, magnesium stearate and colorants or pigments such as titanium dioxide or iron oxide.

According to one embodiment, the composition exhibits a release profile characterized in that at least 40 %, particularly at least 50 %, more preferably at least 60 %, most preferably at least 70 % by weight of the active agent are released within a period of 30 minutes under non-sink conditions (USP paddle, 75 rpm, 900 mL acetate buffer pH 4.5).

According to another embodiment, the composition exhibits a release profile characterized in that at least 80 %, particularly at least 90 %, more preferably at least 95 %, most preferably at least 97 % by weight of the active agent are released within a period of 30 minutes under sink conditions (USP paddle, 75 rpm, 900 mL acetate buffer pH 4.5 containing 0.5 % sodium lauryl sulfate).

It has surprisingly been found that the solid pharmaceutical composition exhibits a dissolution profile and bioavailability that is at least comparable to that of known rivaroxaban formulations, particularly formulations comprising rivaroxaban in hydrophilized form, while allowing for substantially less complex and expensive preparation.

Another advantage is that the solid pharmaceutical composition can be prepared in the absence of solvent, which results in a lower consumption of energy and which is more environmentally friendly.

The composition can be packaged into various kinds of containers. Thus, also disclosed is a packaged pharmaceutical composition comprising the solid pharmaceutical composition described above packaged in a container made of glass or polypropylene with or without desiccant or in a blister made of single or multiple polymer and/or aluminium layers. According to a particular aspect, the packaged composition is packaged with an atmosphere having a reduced oxygen concentration such as below 15 vol.-%, particularly below 10 vol.-%, and more preferably below 5 vol.-% oxygen. According to a particularly preferred embodiment, the composition is packaged with a nitrogen atmosphere.

The invention relates to a process for the preparation of a solid pharmaceutical composition comprising rivaroxaban, or a pharmaceutically acceptable salt or solvate thereof, the process comprising
(a) providing a mixture comprising rivaroxaban, or a pharmaceutically acceptable salt or solvate thereof, and at least one pharmaceutically acceptable excipient comprising (i) hot melt granulation of rivaroxaban with at least one excipient and optionally mixing the obtained granulate with additional excipients or (ii) hot melt extrusion of rivaroxaban with at least one excipient, milling the extrudate and optionally mixing the milled extrudate with additional excipients; and
(b) converting said mixture to a solid pharmaceutical composition, preferably by compressing the mixture to give a tablet;
wherein said process is performed at a temperature of not more than 150°C and wherein the at least one excipient used in step (a) comprises at least one low-melting binder having a melting point below 120 °C which is selected from poloxamers, polyethylene glycols having an average molecular weight of below 15,000, macrogol glycerides and esters of glycerol with fatty acids having 10 to 24 carbon atoms.

It is preferred that the process according to the invention is performed under dry conditions. The term "dry conditions" as used herein refers to a process involving less than 10 %, particularly less than 5 %, and most preferably less than 1 % of water or organic solvents by weight of the solid components used in the process. It is particularly preferred that the process according to the invention is carried out without using any water or organic solvents.

The process is performed at a temperature well below the melting temperature of rivaroxaban. In particular, the process is performed at a temperature of not more than 150 °C, more preferably not more than 100 °C, and most preferably not more than 80 °C.

The process according to the invention preferably comprises single-stage or multi-stage micronization of rivaroxaban to obtain rivaroxaban in micronized form with d₉₀ < 60 µm, preferably d₉₀ < 40 µm, and more preferably d₉₀ < 30 µm. Preferably, micronization of rivaroxaban is performed in the dry or wet state. Dry state milling can be achieved by using dry mills, such as cutting mills, pin/cage mills, hammer mills, jet mills, fluidized bed jet mills, ball mills and roller mills, particularly jet mills. Wet state milling can be achieved by using wet mills, such as colloid mills, ultra sound processors, high pressure homogenizers, wet pearl mills and agitated ball mills, particularly high pressure homogenizers. The liquid used in wet state micronization is typically water. It is particularly preferred that micronized rivaroxaban is used in step (a).

According to one aspect, step (a) comprises dry mixing rivaroxaban with at least one excipient.

According to another aspect, step (a) comprises dry granulation of rivaroxaban with at least one excipient and optionally mixing the obtained granulate with additional excipients. Dry granulation can be performed for instance using roller or slugging compaction and subsequently milling the obtained compact into granules.

According to another aspect, step (a) comprises co-milling of rivaroxaban with at least one excipient and optionally mixing the obtained co-milled mixture with additional excipients. Preferably, the rivaroxaban is co-milled with at least one hydrophilic excipient. Suitable hydrophilic excipients include monosaccharides and oligosaccharides such as glucose, fructose, saccharose, lactose (anhydrous and monohydrate), raffinose, trehalose and dextrates and sugar alcohols such as mannitol, sorbitol, maltitol, xylitol and lactitol. Lactose and sugar alcohols such as mannitol or sorbitol are particularly preferred. In another aspect, rivaroxaban can be co-milled with disintegrant and/or surfactant, such as copovidon and/or sodium dodecyl sulfate, sucrose palmitate or poloxamers. Rivaroxaban and the at least one excipient are preferably co-milled in a ratio of 10:1 - 1:10, particularly 5:1 - 1:8, preferably 1:1 - 1:5, more preferably 1:1.1 - 1:4, most preferably 1:2 - 1:3. Co-milling can be performed for instance using a jet mill. The rivaroxaban, the at least one excipient and/or the co-milled mixture can further be micronized as described above. The mixing of the co-milled mixture with additional excipients may preferably be performed by dry mixing or dry granulation as described immediately above.

In another aspect, rivaroxaban is
1)
   i) micronized or
   ii) co-micronized with at least one excipient or
   iii) co-milled or co-grinded with at least one excipient,
2) the micronized or milled product obtained by 1 i), 1 ii) or 1 iii) is further mixed with at least one excipient, and
3) the product obtained by 2) is agglomerated or granulated by wet granulation without preparing a solution or suspension of rivaroxaban in the granulation liquid.

The average particle size of the agglomerated or granulated particles can be in the range of 20 to 300 µm, preferably 30 to 200 µm. The agglomeration and granulation is used to improve physical properties, such as flowability and/or compressibility of the product.

According to one embodiment, the at least one excipient comprises a liquid or semi-solid waxy surfactant as defined above. Preferably, the at least one excipient used in step (a) comprises a surfactant/filler dispersion. The surfactant/filler dispersion preferably comprises at least one surfactant and at least one filler in a ratio of from 10:1-1:100, particularly 5:1 - 1:50, preferably 2:1 - 1:10, more preferably 1:1 - 1:5, most preferably 1:2 - 1:10.

The surfactant/filler dispersion is typically prepared by a method wherein a surfactant or a mixture of surfactants is finely dispersed with filler and optionally further excipients. Preparation of the surfactant/filler dispersion is typically carried out in the absence of rivaroxaban.

According to one particular method for preparing the surfactant/filler dispersion, the surfactant is dissolved in an organic solvent and the filler is placed into a high shear mixer. The dissolved surfactant is poured onto the filler under stirring and mixed, and the mixture is dried in a fluid bed dryer in order to obtain a solid powdered surfactant/filler dispersion. Alternatively, a liquid or melted surfactant can be simply mixed with a suitable filler for instance in a high-shear mixer.

The obtained surfactant/filler dispersion is mixed with rivaroxaban and optionally other excipients according to any one of the aspects described above. For instance, rivaroxaban may be dry mixed or dry granulated with the surfactant/filler dispersion and optionally further excipients. According to another aspect, rivaroxaban is co-milled with at least one excipient, particularly at least one hydrophilic excipient, and the co-milled mixture is further dry mixed or dry granulated with the surfactant/filler dispersion and optionally further excipients.

According to one embodiment of the invention, step (a) comprises hot melt granulation of rivaroxaban with at least one excipient and optionally mixing the obtained granulate with additional excipients. Hot melt granulation can be performed by granulating rivaroxaban with at least one excipient in a granulating machine such as a high-shear mixer at a temperature of at least the melting point of at least one excipient. Preferably, rivaroxaban is granulated with at least one low-melting binder having a melting point below 120 °C, particularly below 100 °C, and most preferably below 80 °C, at a temperature of at least the melting point of said binder, more particularly at a temperature of at least the melting point of said binder and below the melting temperature of rivaroxaban, the temperature at which the process is performed being not more than 150°C. Preferred low-melting binders are those defined above for the first aspect of the disclosure.

Hot melt granulation can also be performed by heating at least one excipient to at least its melting point, dispersing rivaroxaban and optionally further excipients in the molten excipient and spraying the dispersion onto at least one further excipient. Preferably, the molten excipient is a low-melting binder as described above. In particular, rivaroxaban and a surfactant can be dispersed in molten binder and the dispersion sprayed onto a mixture of filler and optionally disintegrant. The obtained mixture is typically cooled, milled into granules and sieved.

According to another embodiment of the invention, step (a) comprises hot melt extrusion of rivaroxaban with at least one excipient, milling the extrudate and optionally mixing the milled extrudate with additional excipients. Extrusion can be performed using conventional extruders. The obtained extrudate is milled for instance using a jet mill. Preferably, rivaroxaban is extruded with at least one low-melting binder having a melting point below 120 °C, particularly below 100 °C, most preferably below 80 °C, at a temperature of at least the melting point of said binder, more particularly at a temperature of at least the melting point of said binder and below the melting temperature of rivaroxaban, the temperature at which the process is performed being not more than 150°C. Preferred low-melting binders are those defined above for the first aspect of the disclosure.

In step (b), the mixture is preferably compressed to give a tablet. The obtained tablet is optionally film-coated according to coating methods generally known in the art.

It has surprisingly been found that tablets prepared according to the above process exhibit a dissolution profile and bioavailability that is at least comparable to that of known rivaroxaban formulations while offering important advantages in terms of process economy and suitability for industrial application. In particular, processes performed under dry conditions are substantially less complex and expensive than processes involving for instance moist granulation and/or spray-drying. Moreover, processes using hot melt granulation or hot melt extrusion at the relatively low temperatures defined above surprisingly allow for the preparation of tablets having good dissolution and bioavailability properties without subjecting the active agent to temperatures leading to any substantial decomposition thereof.

Further preferred embodiments of the solid pharmaceutical composition with regard to specific components and their amounts as well as with regard to dissolution properties and bioavailability are as described above for the first aspect of the disclosure.

Also disclosed is a solid pharmaceutical composition prepared by the process according to the invention.

The invention will be further illustrated by way of the following examples.

### Examples

### Reference example 1

### Single-stage micronization of rivaroxaban

Rivaroxaban was micronized using single-stage milling. A fluid jet mill with Venturi inlet nozzle operating at a pressure of about 12 bar, a ring pressure of about 11 bar and a dosing mass flow of 20-30 g/min was used (Jetmill MC50, Jet Pharma S.A.). The resultant rivaroxaban was determined to have a particle size distribution of d₉₀ < 15 µm.

The specific surface area of the rivaroxaban was measured by a gas sorption system based on the nitrogen adsorption, using the 6-point Brunauer, Emmett and Teller (BET) method. The measurement showed a change from 0.4 m²/g before milling to 7 m²/g after micronization.

### Reference example 2

### Multi-stage micronization of rivaroxaban

Rivaroxaban was micronized using multi-stage milling. A fluid jet mill with Venturi inlet nozzle operating at a pressure of about 6 bar, a ring pressure of about 4 bar and a dosing mass flow of 20-30 g/min was used in the first stage (Jetmill MC50, Jet Pharma S.A.). The resultant rivaroxaban was determined to have a particle size distribution of d₉₀ < 50 µm.

Subsequently, the rivaroxaban was micronised in a second stage with the Venturi inlet nozzle operating at a pressure of about 12 bar, a ring pressure of about 11 bar and a dosing mass flow of 20-30 g/min (Jetmill MC50, Jet Pharma S.A.). The resultant rivaroxaban was determined to have a particle size distribution of d₉₀ < 10 µm.

### Reference example 2A

### Micronization of rivaroxaban using wet mill

Rivaroxaban was micronized using a wet high pressure homogenizer. Rivaroxaban (30 g) was suspended in 300 g of purified water. The thus prepared suspension was passed through a high pressure homogenizer (APV 2000) at 1800 bar pressure (one pass). The obtained micronized suspension of rivaroxaban was dried using a laboratory spray-drying apparatus (Buchi). The resultant rivaroxaban was determined to have a particle size distribution of d₉₀ < 15 µm.

### Reference example 3

### Co-milling of rivaroxaban together with hydrophilic excipient

50 g of rivaroxaban and 100 g of sorbitol were co-milled using an air-jet mill with an inlet air pressure of about 9 bar and a milling pressure of about 8.5 bar. The resultant mixture of co-milled rivaroxaban and hydrophilic excipient was micronized as described above in Reference examples 1 or 2.

### Reference example 4

### Preparation of tablets by direct compression

Tablet compositions were prepared as follows:

| | **4-1** | **4-2** | **4-3** | **4-4** | **4-5** | **4-6** | **4-7** |
|---|---|---|---|---|---|---|---|
| Rivaroxaban | 10.0 | 10.0 | 10.0 | 10.0 | 10.0 | 10.0 | 10.0 |
| Sodium lauryl sulfate | 2.0 | 2.0 | 2.0 | 2.0 | 2.0 | 2.5 | 2.5 |
| Mannitol | 42.5 | 42.5 | | | | | |
| Lactose spray-dried | | | 42.5 | | 38.5 | 43.9 | 43.9 |
| Cellulose, microcrystalline | 30.0 | | | | 30.0 | 40.0 | 30.0 |
| Calcium hydrogen phosphate | | 30.0 | 30.0 | 68.5 | | | |
| Citric acid | | | | | | | 10.0 |
| Crospovidone | | | | 4.0 | | | |
| Croscarmellose | | | | | 4.0 | 3.0 | 3.0 |
| Magnesium stearate | 0.5 | 0.5 | 0.5 | 0.5 | 0.5 | 0.6 | 0.6 |
| Total mass (mg) | 85.0 | 85.0 | 85.0 | 85.0 | 85.0 | 100.0 | 100.0 |

Micronized rivaroxaban according to Reference example 1 was dry-mixed with the other excipients. The resulting mixture was directly compressed to form tablets.

### Reference example 4A

### Preparation of tablets by direct compression using liquid or semi-solid waxy surfactant

### a) Preparation of surfactant/filler dispersion

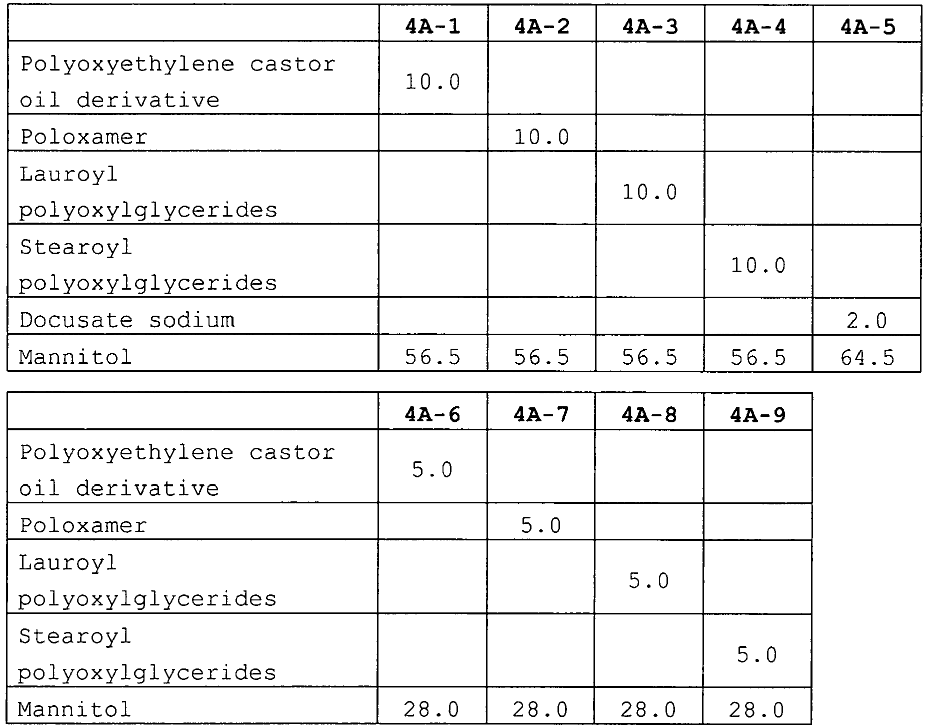

Surfactant (polyoxyethylene castor oil derivative, poloxamer, lauroyl polyoxylglycerides, stearoyl polyoxylglycerides or docusate sodium) was dissolved in an organic solvent such as ethanol, methanol, ethyl acetate or acetone. Filler (mannitol) was placed into a high shear mixer. The dissolved surfactant was poured onto the filler under stirring and mixed, and the mixture was dried in a fluid bed dryer in order to obtain a solid powdered surfactant/filler dispersion.

### b) Preparation of tablet compositions

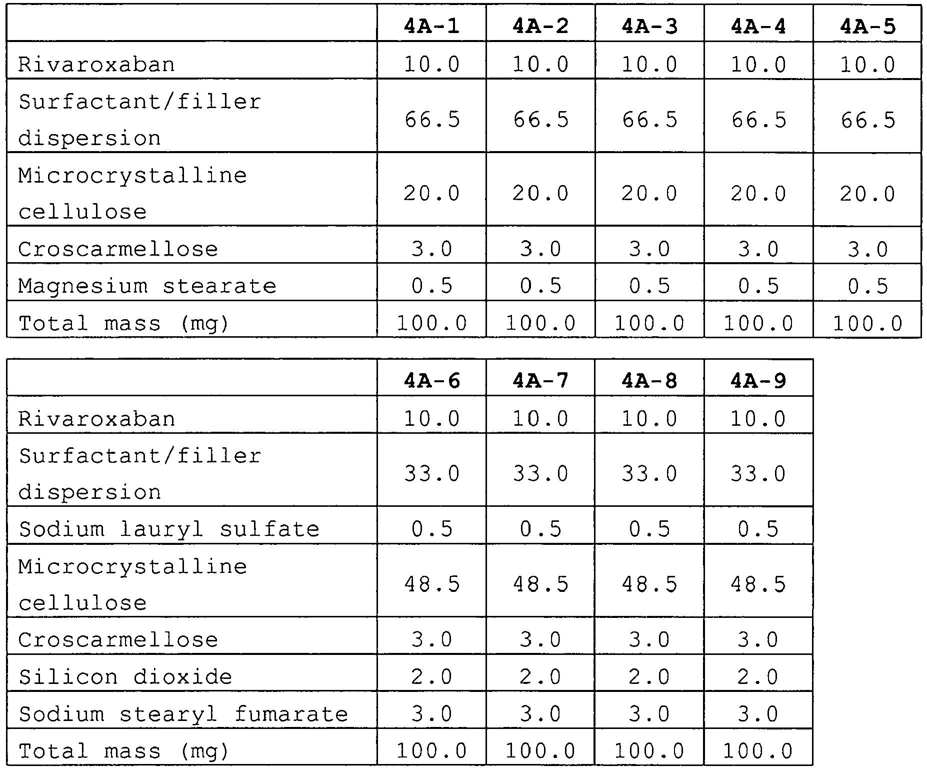

The obtained solid powdered surfactant/filler dispersion was dry mixed in a high shear mixer with rivaroxaban and the remaining excipients, for instance microcrystalline cellulose, croscarmellose and magnesium stearate, in order to obtain a ready-to-compress mixture. This mixture was then compressed into tablets. Rivaroxaban micronized according to Reference example 1 with d₉₀ = 13.2 µm was used in these experiments.

### Reference example 4B

### Preparation of tablets by direct compression using liquid or semi-solid waxy surfactant

### a) Preparation of surfactant/filler dispersion

| | **4B-1** | **4B-2** | **4B-3** | **4B-4** |
|---|---|---|---|---|
| Polysorbate | 10.0 | 3.0 | | |
| Polyoxyethylene castor oil derivative | | | 10.0 | 3.0 |
| Medium-chain triglycerides | | 7.0 | | 7.0 |
| Silicon dioxide | 5.0 | 5.0 | 5.0 | 5.0 |

Surfactant (polysorbate, polyoxyethylene castor oil derivative and/or medium-chain triglycerides) was warmed to about 33 °C and mixed with silicon dioxide in a high-shear mixer in order to prepare a solid powdered surfactant/filler dispersion.

### b) Preparation of tablet compositions

| | **4B-1** | **4B-2** | **4B-3** | **4B-4** |
|---|---|---|---|---|
| Rivaroxaban | 10.0 | 10.0 | 10.0 | 10.0 |
| Surfactant/filler dispersion | 15.0 | 15.0 | 15.0 | 15.0 |
| Mannitol | 51.5 | 51.5 | 51.5 | 51.5 |
| Microcrystalline cellulose | 20.0 | 20.0 | 20.0 | 20.0 |
| Croscarmellose | 3.0 | 3.0 | 3.0 | 3.0 |
| Sodium stearyl fumarate | 0.5 | 0.5 | 0.5 | 0.5 |
| Total mass (mg) | 100.0 | 100.0 | 100.0 | 100.0 |

The obtained solid powdered surfactant/filler dispersion was dry mixed in a high shear mixer with rivaroxaban, microcrystalline cellulose, croscarmellose and magnesium stearate in order to obtain a ready-to-compress mixture. This mixture was then compressed into tablets. Rivaroxaban micronized according to Reference example 1 with d₉₀ = 13.2 µm was used in these experiments.

### Reference example 5

### Preparation of tablets by dry granulation

Tablet compositions were prepared as follows:

| | **5-1** | **5-2** | **5-3** | **5-4** | **5-5** |
|---|---|---|---|---|---|
| Rivaroxaban | 10.0 | 10.0 | 10.0 | 10.0 | 10.0 |
| Sodium lauryl sulfate | 2.0 | 2.0 | 2.0 | 2.0 | 2.0 |
| Mannitol | 42.5 | 42.5 | | | |
| Lactose spray-dried | | | 42.5 | | 38.5 |
| Cellulose, microcrystalline | 30.0 | | | | 30.0 |
| Calcium hydrogen phosphate | | 30.0 | 30.0 | 68.5 | |
| Crospovidone | | | | 4.0 | |
| Croscarmellose | | | | | 4.0 |
| Magnesium stearate | 0.5 | 0.5 | 0.5 | 0.5 | 0.5 |
| Total mass (mg) | 85.0 | 85.0 | 85.0 | 85.0 | 85.0 |

Rivaroxaban was mixed with the other excipients, except Magnesium stearate, sieved and mixed again. The mixture was dry granulated with a roller-compactor using a Fitzpatrik laboratory machine. The resulting compact was milled into granules, mixed with Magnesium stearate and compressed into tablets.

### Reference example 5A

### Preparation of tablets by co-milling in combination with direct compression or dry granulation

### a) Preparation of co-milled rivaroxaban/filler material

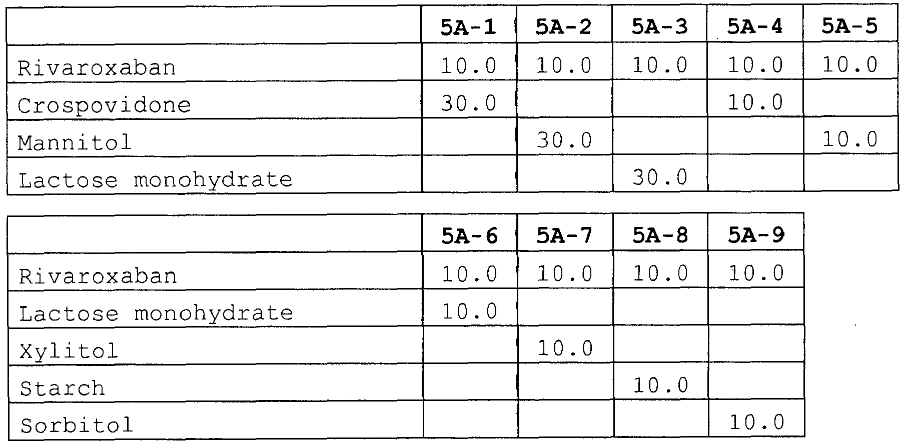

Rivaroxaban was co-milled with filler (crospovidone, mannitol, lactose monohydrate, xylitol, starch or sorbitol) using a fluid jet mill. Rivaroxaban with a particle size distribution d₉₀ < 15 µm was obtained.

### b) Preparation of tablet compositions

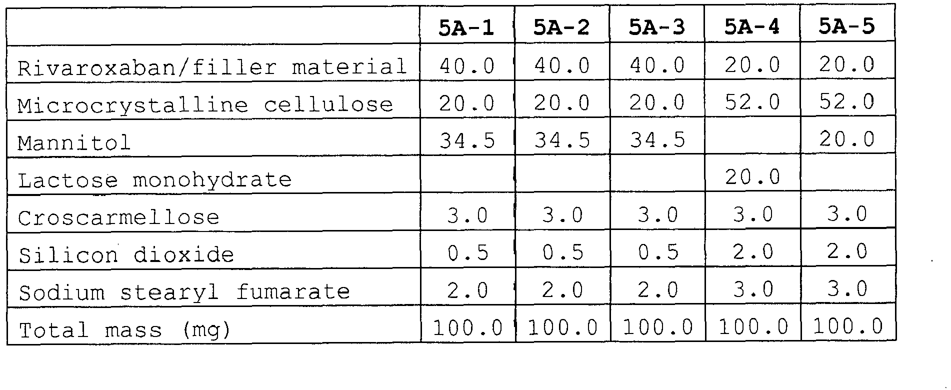

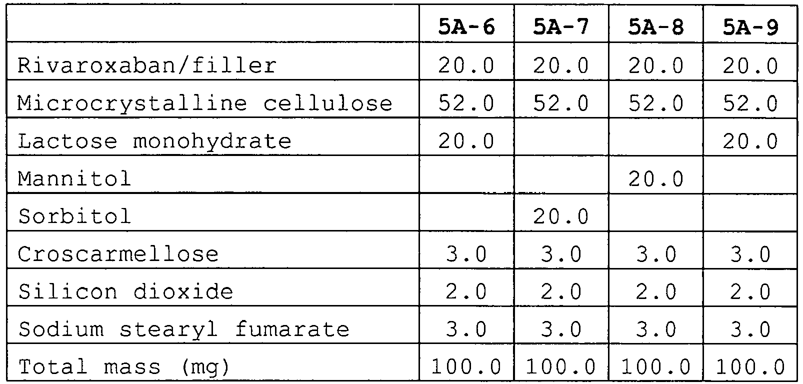

### Alternative mode of preparation 1:

The obtained co-milled rivaroxaban/filler material was dry mixed in a high shear mixer with the remaining excipients, for instance microcrystalline cellulose, mannitol, croscarmellose, silicon dioxide and sodium stearyl fumarate, in order to prepare a ready-to-compress mixture. This mixture was then compressed into tablets.

### Alternative mode of preparation 2:

The obtained co-milled rivaroxaban/filler material was dry mixed in a high shear mixer with the remaining excipients except silicon dioxide and sodium stearyl fumarate, for instance microcrystalline cellulose, mannitol and croscarmellose, in order to prepare a mixture. This mixture was roller compacted (dry-granulated) with an appropriate roller compaction machine. The resulting material was mixed with silicon dioxide and sodium stearyl fumarate in order to obtain a ready-to-compress mixture. This mixture was then compressed into tablets.

### Reference example 5B

### Preparation of tablets by co-milling in combination with preparation of tablets with liquid or semi-solid waxy surfactants

### a) Preparation of co-milled rivaroxaban/filler material

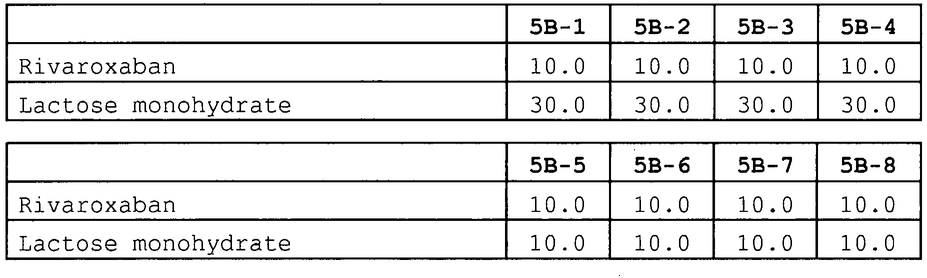

Rivaroxaban was co-milled with a filler (lactose monohydrate) using a fluid jet mill. Rivaroxaban with a particle size distribution d₉₀ = 11.8 µm was obtained.

### b) Preparation of surfactant/filler dispersion

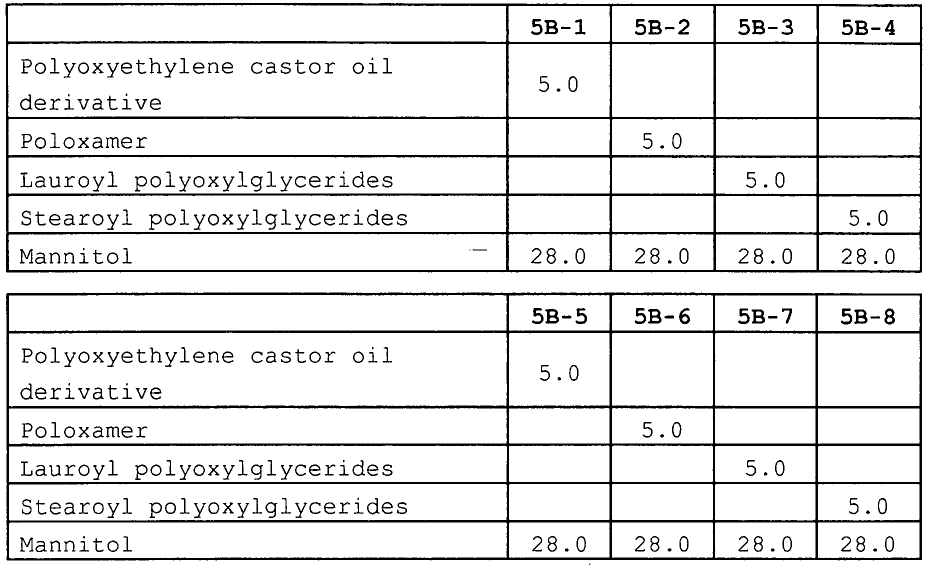

Solid powdered surfactant/filler dispersions were prepared according to Reference example 4A.

### c) Preparation of tablet compositions

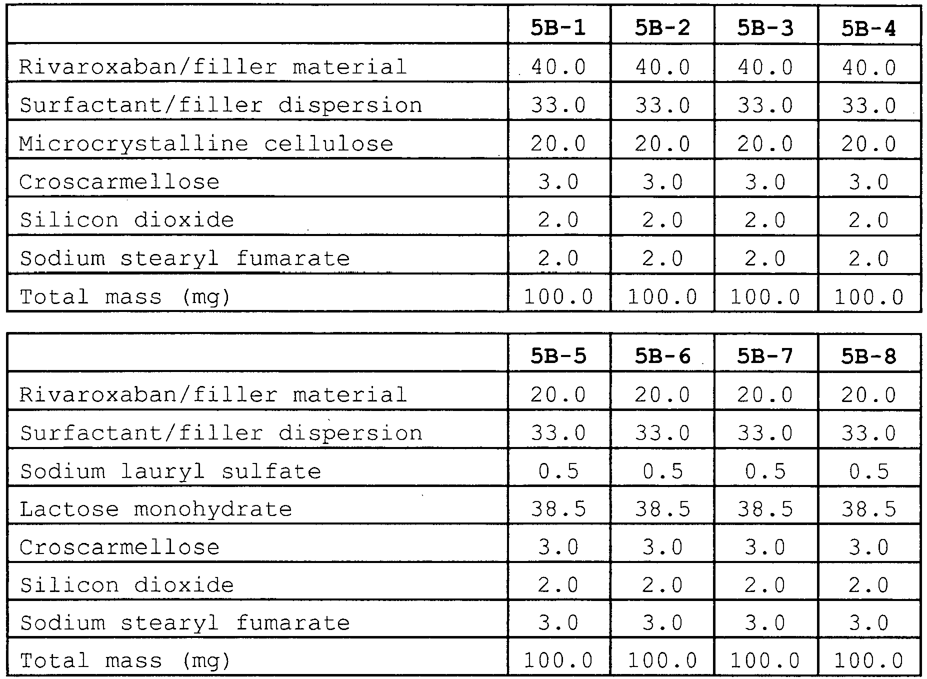

The obtained co-milled rivaroxaban/filler material was dry mixed with solid powdered surfactant/filler dispersion together with the remaining excipients, for instance microcrystalline cellulose, croscarmellose, silicon dioxide and sodium stearyl fumarate, using a high shear mixer. This mixture was then compressed into tablets.

### Reference example 5C

### Preparation of tablets by two-stage co-milling

### a) Preparation of micronized rivaroxaban/filler mixture

| | |
|---|---|
| Rivaroxaban | 10.0 |
| Lactose monohydrate | 30.0 |

Rivaroxaban was micronized using a fluid jet mill according to Reference example 1. Filler (lactose monohydrate) was micronized with the same fluid jet mill under the same conditions. Micronized rivaroxaban was mixed with the micronized filler.

Subsequently, the obtained rivaroxaban/lactose monohydrate mixture was micronized in a second stage as described for Reference example 2. The resultant rivaroxaban was determined to have a particle size distribution of d₉₀ = 6.8 µm.

### b) Preparation of tablet compositions

| | |
|---|---|
| Rivaroxaban/filler | 40.0 |
| Microcrystalline cellulose | 20.0 |
| Mannitol | 34.5 |
| Croscarmellose | 3.0 |
| Silicon dioxide | 0.5 |
| Sodium stearyl fumarate | 2.0 |
| Total mass (mg) | 100.0 |

Using the micronized rivaroxaban/filler mixture obtained above, tablets were prepared as described for Reference example 5A.

### Example 6

### Preparation of tablets by hot melt granulation

Tablet compositions were prepared as follows:

| | **6-1** | **6-2** | **6-3** |
|---|---|---|---|
| Rivaroxaban | 10.0 | 10.0 | 10.0 |
| Polyethylene glycol (e.g. PEG 6000) | 15.0 | | |
| Poloxamer (e.g. Poloxamer 188) | | 15.0 | 15.0 |
| Mannitol | 41.5 | 43.5 | |
| Maize starch | | | 43.5 |
| Crospovidone | 8.0 | 6.0 | 6.0 |
| Cellulose, microcrystalline | 10.0 | 10.0 | 10.0 |
| Colloidal silicon dioxide | 0.5 | 0.5 | 0.5 |
| Total mass (mg) | 85.0 | 85.0 | 85.0 |

Rivaroxaban was mixed with the other excipients (except colloidal silicon dioxide) in a high-shear granulation machine at a temperature of about 55-70 °C for about 5 to 15 minutes. The mixture was cooled down to ambient temperature and mixed with colloidal silicon dioxide. The resulting mixture was compressed into tablets.

### Example 6A

### Preparation of tablets by hot melt granulation

Tablet compositions were prepared as follows:

| | **6A-1** | **6A-2** | **6A-3** | **6A-4** |
|---|---|---|---|---|
| Rivaroxaban | 10.00 | 10.00 | 10.00 | 10.00 |
| Sodium lauryl sulfate | 0.50 | 0.50 | 0.50 | 0.50 |
| Polyethylene glycol | 11.25 | | | |
| Poloxamer | | 11.25 | | |
| Lauroyl polyoxylglycerides | | | 11.25 | |
| Stearoyl polyoxylglycerides | | | | 11.25 |
| Polysorbate | 1.20 | | | |
| Mannitol | 56.30 | 57.50 | 57.50 | 57.50 |
| Microcrystalline cellulose | 10.00 | 10.00 | 10.00 | 10.00 |
| Croscarmellose | 10.00 | 10.00 | 10.00 | 10.00 |
| Silicon dioxide | 0.50 | 0.50 | 0.50 | 0.50 |
| Sodium stearyl fumarate | 0.25 | 0.25 | 0.25 | 0.25 |
| Total mass (mg) | 100.0 | 100.0 | 100.0 | 100.0 |

Rivaroxaban was micronized with a fluid jet mill according to Reference example 1. The resultant Rivaroxaban was determined to have a particle size distribution of d₉₀ = 13.7 µm.

The micronized rivaroxaban was mixed with the other excipients, except colloidal silicon dioxide and sodium stearyl fumarate, in a high-shear granulation machine at a temperature of about 55-70 °C for about 5 to 15 minutes. The mixture was cooled down to ambient temperature and mixed with colloidal silicon dioxide and sodium stearyl fumarate. The resulting mixture was compressed into tablets.

Results of dissolution profile measurement for the tablet composition according to Example 6A-2 were as follows (USP paddle, 75 rpm, 900 mL acetate buffer pH 4.5, 37 °C, 1 tablet containing 10 mg of rivaroxaban):

| | | | | |
|---|---|---|---|---|
| Time elapsed [min] | 15 | 30 | 60 | 90 |
| % active agent released | 65 | 71 | 75 | 78 |

### Reference example 7

### Preparation of tablets by granulation with melted binder

Tablet compositions were prepared as follows:

| | **7-1** | **7-2** |
|---|---|---|
| | | |
| Rivaroxaban | 10.0 | 10.0 |
| Gelucire® 50/14 | 10.0 | |
| Poloxamer 188 | | 10.0 |
| Mannitol (Pearlitol 200 SD) | 40.0 | 40.0 |
| Sodium lauryl sulfate | 1.0 | 1.0 |
| Crospovidone | 2.0 | 1.5 |
| | | |
| Avicel PH 200 | 15.0 | 15.0 |
| Mannitol (Pearlitol 200 SD) | 13.5 | 15.5 |
| Crospovidone | 6.0 | 4.5 |
| Aerosil 200 | 1.0 | 1.0 |
| Magnesium stearate | 1.5 | 1.5 |
| | | |
| Total mass (mg) | 100.0 | 100.0 |

Rivaroxaban and sodium lauryl sulfate were dispersed in the melted binder (Gelucire® or Poloxamer). The dispersion was sprayed onto a mixture of a first amount of mannitol (Pearlitol 200 SD) and crospovidone using adapted laboratory fluid bed equipment Glatt GPCG3. The obtained dispersion was cooled, milled and sieved through a 1 mm sieve. The sieved dispersion was mixed with Avicel and the remaining amounts of mannitol and crospovidone and finally with Aerosil and magnesium stearate and then compressed into tablets.

## Claims

1. A process for the preparation of a solid pharmaceutical composition comprising rivaroxaban, or a pharmaceutically acceptable salt or solvate thereof, the process comprising
(a) providing a mixture comprising rivaroxaban, or a pharmaceutically acceptable salt or solvate thereof, and at least one pharmaceutically acceptable excipient comprising (i) hot melt granulation of rivaroxaban with at least one excipient and optionally mixing the obtained granulate with additional excipients or (ii) hot melt extrusion of rivaroxaban with at least one excipient, milling the extrudate and optionally mixing the milled extrudate with additional excipients; and
(b) converting said mixture to a solid pharmaceutical composition, preferably by compressing the mixture to give a tablet;
wherein said process is performed at a temperature of not more than 150 °C and wherein the at least one excipient used in step (a) comprises at least one low-melting binder having a melting point below 120 °C which is selected from poloxamers, polyethylene glycols having an average molecular weight of below 15,000, macrogol glycerides and esters of glycerol with fatty acids having 10 to 24 carbon atoms.

2. The process according to claim 1, which is performed at a temperature of not more than 100 °C, and most preferably not more than 80 °C.

3. The process according to claim 1 or 2, which is performed without using water or organic solvents.

4. The process according to any one of claims 1 to 3, which further comprises micronization of rivaroxaban, particularly using a jet mill or high pressure homogenizer.

5. The process according to any one of claims 1 to 4, wherein the at least one excipient used in step (a) comprises at least one low-melting binder having a melting point below 100 °C, and most preferably below 80 °C.

6. The process according to any one claims 1 to 5, wherein said composition comprises 1 to 40 wt.-%, particularly 1 to 30 wt.-%, more preferably 5 to 25 wt.-%, and most preferably 10 to 20 wt.-% of low-melting binder.

7. The process according to any one of claims 1 to 6, wherein said low-melting binder is selected from poloxamers, polyethylene glycols having an average molecular weight in the range of 1,500 to 10,000, macrogol glycerides and esters of glycerol with fatty acids having 10 to 24 carbon atoms.

8. The process according to any one of claims 1 to 7, wherein the at least one excipient used in step (a) comprises a surfactant having a melting point of less than 120 °C, more preferably less than 80 °C and most preferably less than 65 °C.

9. The process according to any one of claims 1 to 8, wherein said composition comprises 0.5 to 30 wt.-%, more preferably 1 to 20 wt.-%, particularly 2 to 15 wt.-%, more preferably 3 to 10 wt.-%, and most preferably 4 to 8 wt.-% of surfactant.

10. The process according to claim 8 or 9, wherein said surfactant is selected from docusate sodium, sorbitan fatty acid esters, polysorbates, polyoxyethylene alkyl ethers, poloxamers, medium-chain triglycerides, polyoxylglycerides, polyoxyethylene castor oil derivatives and mixtures thereof.

11. The process according to any one of claims 1 to 10, wherein said composition comprises at least 35 wt.-%, particularly 35 to 90 wt.-%, more particularly 35 to 80 wt.-%, preferably 40 to 70 wt.-%, more preferably 40 to 60 wt.-%, and most preferably 45 to 55 wt.-% of at least one water-soluble filler preferably selected from lactose, mannitol, sorbitol, xylitol and mixtures thereof.

12. The process according to any one of claims 1 to 11, wherein said composition comprises water-soluble filler and water-insoluble filler in a weight ratio of 1:10 to 30:1, particularly 1:1 to 20:1, preferably 1:1 to 10:1, more preferably 2:1 to 8:1, and most preferably 3:1 to 5:1, wherein preferably the water-soluble filler is selected from lactose, mannitol, sorbitol, xylitol and mixtures thereof and/or the water-insoluble filler is selected from microcrystalline cellulose, powdered cellulose, siliconized microcrystalline cellulose, silicon dioxide, crospovidone, croscarmellose sodium, calcium hydrogen phosphate, calcium carbonate, calcium lactate and mixtures thereof.

13. The process according to any one of claims 1 to 12, wherein said composition comprises 1 to 20 wt.-%, particularly 2 to 10 wt.-%, more preferably 3 to 8 wt.-%, and most preferably 4 to 7 wt.-% of disintegrant.

14. The process according to any one of claims 1 to 13, wherein said composition comprises
(a) 0.1 to 60 wt.-%, particularly 0.5 to 40 wt.-%, more preferably 1 to 20 wt.-%, and most preferably 5 to 15 wt.-% of rivaroxaban or a pharmaceutically acceptable salt or solvate thereof;
(b) 30 to 99 wt.-%, particularly 50 to 95 wt.-%, more preferably 70 to 90 wt.-%, and most preferably 80 to 87 wt.-% of filler;
(c) 0.5 to 40 wt.-%, particularly 1 to 20 wt.-%, more preferably 2 to 10 wt.-%, and most preferably 3 to 7 wt.-% of binder;
(d) 0 to 20 wt.-%, particularly 2 to 10 wt.-%, more preferably 3 to 8 wt.-%, and most preferably 4 to 7 wt.-% of disintegrant;
(e) 0 to 30 wt.-%, particularly 1 to 10 wt.-%, more preferably 1.5 to 5 wt.-%, and most preferably 2 to 3 wt.-% of surfactant;
(f) 0 to 10 wt.-%, particularly 0.25 to 5 wt.-%, and more preferably 0.5 to 2 wt.-% of lubricant;
(g) 0 to 10 wt.-%, particularly 0.25 to 5 wt.-%, and more preferably 0.5 to 2 wt.-% of glidant;
(h) 0 to 1 wt.-%, particularly 0.01 to 0.5 wt.-%, and more preferably 0.05 to 0.2 wt.-% of coloring agent; and
(i) 0 to 15 wt.-%, particularly 1 to 12 wt.-% of organic acid.

15. The process according to any of claims 1 to 14, wherein said composition is in the form of a tablet or capsule or in the form of pellets or granules.

## Patentansprüche

1. Verfahren zur Herstellung einer festen pharmazeutischen Zusammensetzung, die Rivaroxaban, ein pharmazeutisch annehmbares Salz oder Solvat davon enthält, bei welchem
(a) eine Mischung zur Verfügung gestellt wird, die Rivaroxaban oder ein pharmazeutisch annehmbares Salz oder Solvat davon und mindestens einen pharmazeutisch annehmbaren Hilfsstoff enthält, indem (i) Rivaroxaban mit mindestens einem Hilfsstoff heißschmelzgranuliert wird und ggf. das erhaltene Granulat mit zusätzlichen Hilfsstoffen gemischt wird oder (ii) Rivaroxaban mit mindestens einem Hilfsstoff heißschmelzextrudiert wird, das Extrudat zerkleinert wird und ggf. das zerkleinerte Extrudat mit zusätzlichen Hilfsstoffen gemischt wird, und
(b) die Mischung zu einer festen pharmazeutischen Zusammensetzung umgewandelt wird, insbesondere durch Verpressen der Mischung, um eine Tablette zu ergeben,
wobei das Verfahren bei einer Temperatur von nicht mehr als 150°C durchgeführt wird und wobei der in Schritt (a) verwendete mindestens eine Hilfsstoff mindestens ein niedrigschmelzendes Bindemittel mit einem Schmelzpunkt unterhalb von 120°C umfasst, welches ausgewählt ist aus Poloxameren, Polyethylenglykolen mit einem mittleren Molekulargewicht von weniger als 15000, Macrogolglyceriden und Estern von Glycerin mit Fettsäuren mit 10 bis 24 Kohlenstoffatomen.

2. Verfahren nach Anspruch 1, welches bei einer Temperatur von nicht mehr als 100°C und besonders bevorzugt nicht mehr als 80°C durchgeführt wird.

3. Verfahren nach Anspruch 1 oder 2, welches ohne Verwendung von Wasser oder organischen Lösungsmitteln durchgeführt wird.

4. Verfahren nach einem der Ansprüche 1 bis 3, welches außerdem die Mikronisation von Rivaroxaban, insbesondere unter Verwendung einer Strahlmühle oder eines Hochdruckhomogenisators, beinhaltet.

5. Verfahren nach einem der Ansprüche 1 bis 4, bei dem der in Schritt (a) verwendete mindestens eine Hilfsstoff mindestens ein niedrigschmelzendes Bindemittel mit einem Schmelzpunkt unterhalb 100°C und besonders bevorzugt unterhalb 80°C umfasst.

6. Verfahren nach einem der Ansprüche 1 bis 5, bei dem die Zusammensetzung 1 bis 40 Gew.-%, insbesondere 1 bis 30 Gew.-%, weiter bevorzugt 5 bis 25 Gew.-% und besonders bevorzugt 10 bis 20 Gew.-% niedrigschmelzendes Bindemittel enthält.

7. Verfahren nach einem der Ansprüche 1 bis 6, bei dem das niedrigschmelzende Bindemittel ausgewählt ist aus Poloxameren, Polyethylenglykolen mit einem mittleren Molekulargewicht im Bereich von 1500 bis 10000, Macrogolglyceriden und Estern von Glycerin mit Fettsäuren mit 10 bis 24 Kohlenstoffatomen.

8. Verfahren nach einem der Ansprüche 1 bis 7, bei dem der in Schritt (a) verwendete mindestens eine Hilfsstoff ein Tensid mit einem Schmelzpunkt von weniger als 120°C, bevorzugt weniger als 80°C und besonders bevorzugt weniger als 65°C umfasst.

9. Verfahren nach einem der Ansprüche 1 bis 8, bei dem die Zusammensetzung 0,5 bis 30 Gew.-%, insbesondere 1 bis 20 Gew.-%, bevorzugt 2 bis 15 Gew.-%, besonders bevorzugt 3 bis 10 Gew.-% und am meisten bevorzugt 4 bis 8 Gew.-% Tensid enthält.

10. Verfahren nach Anspruch 8 oder 9, bei dem das Tensid ausgewählt ist aus Natriumdocusat, Sorbitanfettsäureestern, Polysorbaten, Polyoxyethylenalkylethern, Poloxameren, mittelkettigen Triglyceriden, Polyoxylglyceriden, Polyoxyethylenrizinusölderivaten und Mischungen davon.

11. Verfahren nach einem der Ansprüche 1 bis 10, bei dem die Zusammensetzung mindestens 35 Gew.-%, insbesondere 35 bis 90 Gew.-%, bevorzugter 35 bis 80 Gew.-%, weiter bevorzugt 40 bis 70 Gew.-%, besonders bevorzugt 40 bis 60 Gew.-% und am meisten bevorzugt 45 bis 55 Gew.-% von mindestens einem wasserlöslichen Füllstoff enthält, der vorzugsweise ausgewählt ist aus Laktose, Mannit, Sorbit, Xylit und Mischungen davon.

12. Verfahren nach einem der Ansprüche 1 bis 11, bei dem die Zusammensetzung wasserlöslichen Füllstoff und wasserunlöslichen Füllstoff in einem Gewichtsverhältnis von 1:10 bis 30:1, insbesondere 1:1 bis 20:1, bevorzugter 1:1 bis 10:1, besonders bevorzugt 2:1 bis 8:1 und am meisten bevorzugt 3:1 bis 5:1 enthält, wobei vorzugsweise der wasserlösliche Füllstoff ausgewählt ist aus Laktose, Mannit, Sorbit, Xylit und Mischungen davon und/oder der wasserunlösliche Füllstoff ausgewählt ist aus mikrokristalliner Cellulose, gepulverter Cellulose, silikonisierter mikrokristalliner Cellulose, Siliciumdioxid, Crospovidon, Natriumcroscarmellose, Calciumhydrogenphosphat, Calciumcarbonat, Calciumlaktat und Mischungen davon.

13. Verfahren nach einem der Ansprüche 1 bis 12, bei dem die Zusammensetzung 1 bis 20 Gew.-%, insbesondere 2 bis 10 Gew.-%, bevorzugter 3 bis 8 Gew.-% und am meisten bevorzugt 4 bis 7 Gew.-% Sprengmittel enthält.

14. Verfahren nach einem der Ansprüche 1 bis 13, bei dem die Zusammensetzung
(a) 0,1 bis 60 Gew.-%, insbesondere 0,5 bis 40 Gew.-%, bevorzugter 1 bis 20 Gew.-% und am meisten bevorzugt 5 bis 15 Gew.-% Rivaroxaban oder ein pharmazeutisch annehmbares Salz oder Solvat davon,
(b) 30 bis 99 Gew.-%, insbesondere 50 bis 95 Gew.-%, bevorzugter 70 bis 90 Gew.-% und am meisten bevorzugt 80 bis 87 Gew.-% Füllstoff,
(c) 0,5 bis 40 Gew.-%, insbesondere 1 bis 20 Gew.-%, bevorzugter 2 bis 10 Gew.-% und am meisten bevorzugt 3 bis 7 Gew.-% Bindemittel,
(d) 0 bis 20 Gew.-%, insbesondere 2 bis 10 Gew.-%, bevorzugter 3 bis 8 Gew.-% und am meisten bevorzugt 4 bis 7 Gew.-% Sprengmittel,
(e) 0 bis 30 Gew.-%, insbesondere 1 bis 10 Gew.-%, bevorzugter 1,5 bis 5 Gew.-% und am meisten bevorzugt 2 bis 3 Gew.-% Tensid,
(f) 0 bis 10 Gew.-%, insbesondere 0,25 bis 5 Gew.-% und besonders bevorzugt 0,5 bis 2 Gew.-% Schmiermittel,
(g) 0 bis 10 Gew.-%, insbesondere 0,25 bis 5 Gew.-% und besonders bevorzugt 0,5 bis 2 Gew.-% Gleitmittel,
(h) 0 bis 1 Gew.-%, insbesondere 0,01 bis 0,5 Gew.-% und besonders bevorzugt 0,05 bis 0,2 Gew.-% Färbemittel und
(i) 0 bis 15 Gew.-%, insbesondere 1 bis 12 Gew.-% organische Säure
enthält.

15. Verfahren nach einem der Ansprüche 1 bis 14, bei dem die Zusammensetzung in Form einer Tablette oder Kapsel oder in Form von Pellets oder Granulatkörnern ist.

## Revendications

1. Procédé en vue de la préparation d'une composition pharmaceutique solide comprenant du rivaroxaban, ou un sel ou solvate pharmacologiquement admissible de ce composé, le procédé comportant les étapes suivantes :
(a) production d'un mélange comprenant du rivaroxaban, ou un sel ou solvate pharmacologiquement admissible de ce composé, et au moins un excipient pharmacologiquement admissible, comprenant (i) la granulation par fusion du rivaroxaban avec au moins un excipient et, en option, le brassage du produit de granulation avec des excipients supplémentaires, ou (ii) l'extrusion à chaud du rivaroxaban avec au moins un excipient, le broyage de l'extrudat et, en option, le brassage de l'extrudat broyé avec des excipients supplémentaires ;
(b) et transformation dudit mélange en une composition pharmaceutique solide, de préférence par compression du mélange pour qu'il forme un comprimé ;
lequel procédé est effectué à une température ne dépassant pas 150°C, et l'excipient au nombre d'au moins un qui est employé dans l'étape (a) comprenant au moins un liant fondant à basse température dont le point de fusion est inférieur à 120°C, choisi parmi les poloxamères, les polyéthylène-glycols de masse moléculaire moyenne inférieure à 15 000, les macrogol-glycérides et les esters de glycérol formés avec des acides gras comportant de 10 à 24 atomes de carbone.

2. Procédé selon la revendication 1, qui est effectué à une température ne dépassant pas 100°C et au mieux ne dépassant pas 80°C.

3. Procédé selon la revendication 1 ou 2, qui est effectué sans utilisation d'eau ou de solvants organiques.

4. Procédé selon l'une quelconque des revendications 1 à 3, qui comprend en outre la micronisation du rivaroxaban, en particulier au moyen d'un broyeur à jet ou d'un homogénéisateur à haute pression.

5. Procédé selon l'une quelconque des revendications 1 à 4, dans lequel l'excipient au nombre d'au moins un qui est employé dans l'étape (a) comprend au moins un liant fondant à basse température dont le point de fusion est inférieur à 100°C et au mieux inférieur à 80°C.

6. Procédé selon l'une quelconque des revendications 1 à 5, dans lequel ladite composition comprend de 1 à 40 % en poids, en particulier 1 à 30 % en poids, encore mieux 5 à 25 % en poids et au mieux 10 à 20 % en poids de liant fondant à basse température.

7. Procédé selon l'une quelconque des revendications 1 à 6, dans lequel ledit liant fondant à basse température est choisi parmi les poloxamères, les polyéthylène-glycols de masse moléculaire moyenne comprise dans l'intervalle allant de 1500 à 10 000, les macrogol-glycérides et les esters de glycérol formés avec des acides gras comportant de 10 à 24 atomes de carbone.

8. Procédé selon l'une quelconque des revendications 1 à 7, dans lequel l'excipient au nombre d'au moins un qui est employé dans l'étape (a) comprend un agent tensioactif dont le point de fusion est inférieur à 120°C, mieux encore inférieur à 80°C et au mieux inférieur à 65°C.

9. Procédé selon l'une quelconque des revendications 1 à 8, dans lequel ladite composition comprend de 0,5 à 30 % en poids, mieux encore 1 à 20 % en poids, en particulier 2 à 15 % en poids, encore mieux 3 à 10 % en poids et au mieux 4 à 8 % en poids d'agent tensioactif.

10. Procédé selon la revendication 8 ou 9, dans lequel ledit agent tensioactif est choisi dans l'ensemble constitué par le docusate sodique, les esters d'acides gras de sorbitane, les polysorbates, les éthers d'alkyle polyéthoxylés, les poloxamères, les triglycérides à chaîne moyenne, les polyoxylglycérides, les dérivés d'huile de ricin polyéthoxylés et les mélanges de tels composés.

11. Procédé selon l'une quelconque des revendications 1 à 10, dans lequel ladite composition comprend au moins 35 % en poids, en particulier 35 à 90 % en poids, plus particulièrement 35 à 80 % en poids, de préférence 40 à 70 % en poids, mieux encore 40 à 60 % en poids et au mieux 45 à 55 % en poids d'au moins une charge hydrosoluble, de préférence choisie dans l'ensemble constitué par le lactose, le mannitol, le sorbitol, le xylitol et les mélanges de tels composés.

12. Procédé selon l'une quelconque des revendications 1 à 11, dans lequel ladite composition comprend une charge hydrosoluble et une charge insoluble dans l'eau en un rapport pondéral valant de 1/10 à 30/1, en particulier 1/1 à 20/1, de préférence 1/1 à 10/1, mieux encore 2/1 à 8/1 et au mieux 3/1 à 5/1, étant entendu que, de préférence, la charge hydrosoluble est choisie dans l'ensemble constitué par le lactose, le mannitol, le sorbitol, le xylitol et les mélanges de tels composés, et/ou la charge insoluble dans l'eau est choisie dans l'ensemble constitué par la cellulose microcristalline, la cellulose en poudre, la cellulose microcristalline siliconée, le dioxyde de silicium, la crospovidone, la croscarmellose sodique, l'hydrogénophosphate de calcium, le carbonate de calcium, le lactate de calcium et les mélanges de tels composés.

13. Procédé selon l'une quelconque des revendications 1 à 12, dans lequel ladite composition comprend de 1 à 20 % en poids, en particulier 2 à 10 % en poids, mieux encore 3 à 8 % en poids et au mieux 4 à 7 % en poids de désintégrant.

14. Procédé selon l'une quelconque des revendications 1 à 13, dans lequel ladite composition comprend :
(a) de 0,1 à 60 % en poids, en particulier 0,5 à 40 % en poids, encore mieux 1 à 20 % en poids et au mieux 5 à 15 % en poids de rivaroxaban, ou d'un sel ou solvate pharmacologiquement admissible de ce composé ;
(b) de 30 à 99 % en poids, en particulier 50 à 95 % en poids, encore mieux 70 à 90 % en poids et au mieux 80 à 87 % en poids de charge ;
(c) de 0,5 à 40 % en poids, en particulier 1 à 20 % en poids, encore mieux 2 à 10 % en poids et au mieux 3 à 7 % en poids de liant ;
(d) de 0 à 20 % en poids, en particulier 2 à 10 % en poids, encore mieux 3 à 8 % en poids et au mieux 4 à 7 % en poids de désintégrant ;
(e) de 0 à 30 % en poids, en particulier 1 à 10 % en poids, encore mieux 1,5 à 5 % en poids et au mieux 2 à 3 % en poids d'agent tensioactif ;
(f) de 0 à 10 % en poids, en particulier 0,25 à 5 % en poids et mieux encore 0,5 à 2 % en poids de lubrifiant ;
(g) de 0 à 10 % en poids, en particulier 0,25 à 5 % en poids et mieux encore 0,5 à 2 % en poids d'agent d'écoulement ;
(h) de 0 à 1 % en poids, en particulier 0,01 à 0,5 % en poids et mieux encore 0,05 à 0,2 % en poids d'agent colorant ;
(i) et de 0 à 15 % en poids, en particulier 1 à 12 % en poids d'acide organique.

15. Procédé selon l'une quelconque des revendications 1 à 14, dans lequel ladite composition se présente sous la forme d'un comprimé ou d'une capsule, ou sous la forme de pellets ou de granulés.
